(19) Europäisches Patentamt / European Patent Office / Office européen des brevets

(11) **EP 3 517 021 B1**

(12) **EUROPÄISCHE PATENTSCHRIFT**

(45) Veröffentlichungstag und Bekanntmachung des Hinweises auf die Patenterteilung:
**14.10.2020 Patentblatt 2020/42**

(51) Int Cl.:
***A61B 3/10*** *(2006.01)*

(21) Anmeldenummer: **18153564.2**

(22) Anmeldetag: **26.01.2018**

(54) **FULL-FIELD-OCT-VERFAHREN UND -SYSTEM ZUM ERZEUGEN EINER ABBILDUNG EINES AUGENHINTERGRUNDS**

FILLING FIELD OCT METHOD AND SYSTEM FOR GENERATING AN IMAGE OF AN EYE FUNDUS

PROCÉDÉ ET SYSTÈME À CHAMP PLEIN OCT PERMETTANT DE GÉNÉRER UNE IMAGE DU FOND DE L' IL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**31.07.2019 Patentblatt 2019/31**

(73) Patentinhaber: **Visotec GmbH**
**23552 Lübeck (DE)**

(72) Erfinder:
• **KOCH, Peter**
  **23558 Lübeck (DE)**
• **HÜTTMANN, Gereon**
  **23564 Lübeck (DE)**
• **SUDKAMP, Helge**
  **23552 Lübeck (DE)**
• **SPAHR, Hendrik**
  **23562 Lübeck (DE)**
• **HILLMANN, Dierck**
  **23552 Lübeck (DE)**
• **MÜNST, Michael**
  **23560 Lübeck (DE)**

(74) Vertreter: **Glawe, Delfs, Moll Partnerschaft mbB von Patent- und Rechtsanwälten Postfach 13 03 91 20103 Hamburg (DE)**

(56) Entgegenhaltungen:
**DE-A1-102015 113 465     US-A1- 2007 038 040**

**Beschreibung**

Die Erfindung betrifft ein Full-Field-OCT-Verfahren und ein Full-Field-OCT-System zum Erzeugen einer Abbildung eines Augenhintergrunds.

Der Augenhintergrund eines menschlichen Auges kann durch optische Kohärenztomografie (OCT) untersucht werden. Es können Bildinformationen über Gewebestrukturen gewonnen werden, die unter der Oberfläche liegen. Bei einer OCT-Messung wird kurzkohärentes Licht in einen Objektstrahlengang und einen Referenzstrahlengang aufgespalten. Der Objektstrahlengang wird auf den Augenhintergrund geleitet. Ein vom Augenhintergrund reflektierter Teil des Objektstrahlengangs wird zur Interferenz mit dem Referenzstrahlengang gebracht. Aus dem Referenzmuster kann eine Information über die Tiefenposition eines Streuzentrums im Gewebe des Augenhintergrunds gewonnen werden.

Bei klassischer OCT-Bildgebung wird das Objekt mit einem OCT-Strahl abgetastet (gescannt), um ein flächiges Bild (En-face-Bild) zu erhalten. Die Full-Field-OCT unterscheidet sich von diesen klassischen OCT-Verfahren dadurch, dass ein Bildsensor verwendet wird, der mit einer Aufnahme eine En-face-Bild aufnimmt.

In DE 10 2015 113 465 A1 ist ein Full-Field-OCT-Verfahren zur Untersuchung des Augenhintergrunds beschrieben, bei dem der Objektstrahlengang und der Referenzstrahlengang auf einem Bildsensor zur Interferenz gebracht werden. Der Referenzstrahlengang trifft unter einem anderen Winkel auf den Bildsensor, so dass aus den Messwerten des Bildsensors eine Intensitätsinformation und eine Phaseninformation abgeleitet werden können. Eine einzelne Aufnahme des Bildsensors ergibt einen En-face-Schnitt des Objekts. Durch Verändern der Länge des optischen Wegs im Referenzstrahlengang können Schichtbilder aus verschiedenen Tiefenpositionen gewonnen werden. Die Schichtbilder können zu einem dreidimensionalen Bild zusammengesetzt werden.

In DE 10 2015 113 465 A1 werden die optischen Elemente im Objektstrahlengang so justiert, dass der Augenhintergrund scharf auf den Bildsensor abgebildet wird. Vor jeder Messung an einem anderen Auge muss der Objektstrahlengang neu justiert werden, um auf dem Bildsensor eine scharfe Abbildung zu erhalten. Der Objektstrahlengang erstreckt sich nämlich durch die brechenden Elemente (Linse, Cornea) des Auges hindurch. Da die Abbildungseigenschaften der brechenden Elemente nicht bei allen Augen gleich sind, würde sich ohne Justieren ein defokusiertes und deswegen unscharfes Bild auf dem Bildsensor ergeben.

Der Erfindung liegt die Aufgabe zugrunde, ein Full-Field-OCT-Verfahren und -System vorzustellen, bei dem der Aufwand bei der Aufnahme eines Bilds des Augenhintergrunds vermindert ist. Ausgehend vom genannten Stand der Technik wird die Aufgabe gelöst mit den Merkmalen der unabhängigen Ansprüche. Vorteilhafte Ausführungsformen sind in den Unteransprüchen angegeben.

Bei dem erfindungsgemäßen Verfahren wird kurzkohärentes Licht ausgesendet. Das kurzkohärente Licht wird in einen Objektstrahlengang und einen Referenzstrahlengang aufgespalten, wobei der Objektstrahlengang auf den Augenhintergrund geleitet wird. Der Referenzstrahlengang und ein von dem Augenhintergrund reflektierter Teil des Objektstrahlengangs werden auf einen Bildsensor geleitet, so dass auf dem Bildsensor eine Interferenz zwischen dem Referenzstrahlengang und dem Objektstrahlengang entsteht. Der Referenzstrahlengang trifft unter einem von dem Objektstrahlengang abweichenden Winkel auf den Bildsensor. Der Referenzstrahlengang trifft vor dem Auftreffen auf den Bildsensor auf ein optisches Korrekturelement, um eine chromatische Abweichung innerhalb des Referenzstrahlengangs zu vermindern. Aus der Aufnahme des Bildsensors werden eine Intensitätsinformation und eine Phaseninformation ermittelt, und es wird ein fokuskorrigiertes Bild des Augenhintergrunds berechnet.

Mit der Erfindung wird also vorgeschlagen, einen Abbildungsfehler bei der Full-Field-OCT-Messung nicht durch Justieren von optischen Elementen im Objektstrahlengang, sondern durch eine Korrekturrechnung auszugleichen. Durch die Erfindung wird es möglich, auf eine Justierung von optischen Elementen des Objektstrahlengangs zu verzichten. Durch diese Erleichterung kann eine Abbildung des Augenhintergrunds auch von solchen Personen aufgenommen werden, die keine Kenntnisse von optischen Strahlengängen haben.

Dass es bei Vorliegen einer Intensitätsinformation und einer Phaseninformation grundsätzlich möglich ist, einen Fokusfehler einer einzelnen Lichtwelle auf rechnerischen Wege zu beseitigen, ist Lehrbuchwissen (J. W. Goodmann: Introduction to Fourier Optics; Schnars U, Jueptner W (2010). Digital Holography (Berlin, Heidelberg: Springer). Eine direkte Anwendung solcher rechnerischer Korrekturverfahren auf Full-Field-OCT-Messungen ist aus folgenden Gründen nicht möglich.

Bei einer Full-Field-OCT-Messung nach DE 10 2015 113 465 A1 wird mit einer Aufnahme ein vollständiges Bild aufgenommen. Im Unterschied zu einer einzelnen Lichtwelle kann also eine ortsaufgelöste Information in die Korrekturberechnung einbezogen werden. Das Berücksichtigen der ortsaufgelösten Information wird erschwert, weil die abweichenden Winkel, unter denen der Objektstrahlengang und der Referenzstrahlengang auf dem Bildsensor auftreffen, in vielen Fällen mit einer chromatischen Abweichung einhergehen. Dies gilt insbesondere, wenn der Objektstrahlengang nicht in der Ebene des Bildsensors fokussiert ist. Durch den Winkel zwischen dem Referenzstrahlengang und dem Objektstrahlengang wird der Interferenzterm mit einer Trägerfrequenz kodiert. Die Trägerfrequenz ist jedoch von der Wellenlänge abhängig, was im Frequenzraum zu einer chromatischen Verschmierung führt. Diese verhindert eine effektive Korrektur.

Die Erfindung hat erkannt, dass die chromatische Verschmierung durch ein optisches Korrekturelement im Referenzstrahlengang aufgehoben werden kann. Bei Anwendung des erfindungsgemäßen Verfahrens ist die rechnerische Beseitigung eines Fokusfehlers nicht mehr durch eine chromatische Verschmierung erschwert.

Im Unterschied zur Full-Field-OCT ist bei der scannenden OCT eine rechnerische Beseitigung eines Fokusfehlers nicht möglich, weil die scannende OCT nach jetzigem Stand der Messtechnik keine Phaseninformation in ausreichender Qualität liefert.

Das erfindungsgemäße Verfahren kann so durchgeführt werden, dass der Objektstrahlengang unter einem rechten Winkel auf den Bildsensor auftrifft. Der Winkel, den der Referenzstrahlengang mit der Arrayebene einschließt, ist dann kein rechter Winkel.

Möglich sind auch Ausführungsformen, bei denen der Objektstrahlengang nicht unter einem rechten Winkel auf den Bildsensor trifft.

Für die Aufspaltung des kurzkohärenten Lichts in den Objektstrahlengang und den Referenzstrahlengang wird ein Strahlenteiler verwendet, durch den das kurzkohärente Licht hindurchtritt. Der Strahlenteiler kann so angeordnet sein, dass der Objektstrahlengang und/oder der Referenzstrahlengang den Strahlenteiler zweimal mit unterschiedlicher Laufrichtung passieren. Der Objektstrahlengang kann von dem Augenhintergrund in Richtung des Strahlenteilers zurückgeworfen werden. Im Referenzstrahlengang kann ein Reflexionselement angeordnet sein, mit dem der Referenzstrahlengang umgelenkt wird, und insbesondere in Richtung des Strahlenteilers zurückgeworfen wird. Der Referenzstrahlengang vor dem Reflexionselement kann von dem Referenzstrahlengang nach dem Reflexionselement separiert sein, so dass es keine Überschneidung zwischen dem bei dem Reflexionselement eintreffenden Strahlengang und dem von dem Reflexionselement ausgehenden Strahlengang gibt. Dies betrifft einen zu dem Reflexionselement benachbarten Abschnitt des Referenzstrahlengangs. Mit größerem Abstand von dem Reflexionselement kann es wieder zu einer Überschneidung der entgegengesetzten Abschnitte des Referenzstrahlengangs kommen. Das Reflexionselement kann ein Prisma sein, insbesondere ein Dachprisma. Eine Ebene, die sich durch die Dachkante des Prismas erstreckt, kann zwischen dem eintreffenden Teil und dem ausgehenden Teil des Referenzstrahlengangs angeordnet sein.

Die Länge des optischen Wegs des Referenzstrahlengangs kann veränderbar sein. Insbesondere kann die Länge des optischen Wegs des Referenzstrahlengangs veränderbar sein, ohne dass zugleich die Länge des optischen Wegs des Objektstrahlengangs verändert wird. Beispielsweise kann es möglich sein, dass Reflexionselement zu verschieben, so dass der Abstand zwischen dem Reflexionselement und dem Strahlenteiler verändert wird. Wird der Abstand zwischen dem Reflexionselement und dem Strahlenteiler vergrößert, so legt der Referenzstrahlengang auf dem Weg vom Strahlenteiler zu dem Reflexionselement und zurück den verlängerten Weg zweimal zurück. Durch Verändern der Länge des optischen Wegs im Referenzstrahlengang kann die Tiefenposition im Gewebe des Augenhintergrunds eingestellt werden, in der Streuzentren entdeckt werden können. Das Verfahren kann so durchgeführt werden, dass in mehreren Tiefenpositionen En-face-Schichtbilder aufgenommen werden. Die Mehrzahl der En-face-Schichtbilder kann zu einer dreidimensionalen Abbildung des Augenhintergrunds zusammengesetzt werden.

Das optische Korrekturelement kann zwischen dem Strahlenteiler und dem Reflexionselement angeordnet sein. Das optische Korrekturelement kann so angeordnet sein, dass der Referenzstrahlengang nur einmal durch das optische Korrekturelement hindurchtritt. Insbesondere kann der Durchtritt durch das optische Korrekturelement erfolgen, wenn der Referenzstrahlengang sich von dem Reflexionselement zu dem Strahlenteiler bewegt.

Das optische Korrekturelement kann ein Transmissionsgitter sein. Im Falle eines Transmissionsgitters kann der Referenzstrahlengang durch das optische Korrekturelement hindurchtreten. Möglich ist auch, das optische Korrekturelement als Reflexionsgitter zu gestalten. In diesem Falle kann der Referenzstrahlengang an dem optischen Korrekturelement reflektiert werden. Das Transmissionsgitter bzw. das Reflexionsgitter kann Gitterlinien umfassen, die senkrecht zu einer Ebene ausgerichtet sind, die der Objektstrahlengang und der Referenzstrahlengang zwischen dem Strahlenteiler und dem Auge bzw. dem Reflexionselement aufspannen.

Das Transmissionsgitter kann so ausgelegt sein, dass es eine Ablenkung des Referenzstrahlengangs zwischen 1° und 5°, vorzugsweise zwischen 2° und 4° bewirkt. Das Transmissionsgitter bzw. Reflexionsgitter kann zwischen 25 und 100 Gitterlinien pro Millimeter, vorzugsweise zwischen 500 und 80 Gitterlinien pro Millimeter aufweisen.

Die Verminderung der chromatischen Abweichung kann sich insbesondere daraus ergeben, dass die Pulsfront des aus dem optischen Korrekturelement auslaufenden Referenzstrahlengangs von der Ausbreitungsrichtung des Referenzstrahlengangs abweicht. Insbesondere kann das optische Korrekturelement so eingestellt sein, dass die Normale der Pulsfront des aus dem optischen Korrekturelement auslaufenden Referenzstrahlengangs einen Winkel mit der Ausbreitungsrichtung des Objektstrahlengangs einschließt, der kleiner ist als der Winkel zwischen der Ausbreitungsrichtung des Referenzstrahlengangs und der Ausbreitungsrichtung des Objektstrahlengangs. Insbesondere kann die Normale der Pulsfront des aus dem optischen Korrekturelement auslaufenden Referenzstrahlengangs parallel zu der Ausbreitungsrichtung des Objektstrahlengangs sein.

Durch einen solchen optischen Aufbau des Full-Field-OCT-Systems, entsteht auf dem Bildsensor ein Interferenzmuster innerhalb der einzelnen Bildelemente (Speckle), aus dem sowohl die Amplitude der Rückstreuung in der entsprechenden Tiefe als auch die zugehörige Phasenlage ermittelt werden kann, siehe DE 10 2015 113 465 A1. Bei einer idealen Abbildung des Objektstrahlengangs, der sich durch die Linse des menschlichen Auges sowie die optischen Elemente des Full-Field-OCT-Aufbaus hindurch erstreckt, wird die von einem Objektpunkt des Augenhintergrunds emittierte Kugelwelle in eine konvergente Kugelwelle umgewandelt, die einen Bildpunkt auf dem Bildsensor bildet.

Der Objektstrahlengang des Full-Field-OCT-Systems kann beispielsweise so eingerichtet sein, dass die ideale Abbildung auf den Bildsensor sich einstellt, wenn ein Objektpunkt des Augenhintergrunds durch die brechenden Elemente des Auges (Linse und Cornea) ins Unendliche abgebildet wird. Die brechenden Elemente des Auges werden nachfolgend gemeinsam als Augenlinse bezeichnet. Bei einer Fehlsichtigkeit des Patienten bildet die Augenlinse nicht aus dem Unendlichen bzw. ins Unendliche ab, sondern in eine Ebene vor oder hinter dem Auge. Für den Objektstrahlengang des Full-Field-OCT-Systems bedeutet eine solche Aberration, dass die Bildebene vor oder hinter dem Bildsensor liegt.

Aus der Fourier-Optik ist bekannt, dass Aberrationen sich als Phasenfehler darstellen lassen. Abbildungsfehler führen zu einer Verformung der Phasenfront des Lichts im Strahlengang. Ein Fokusfehler, also die Abbildung in die falsche Bildebene, lässt sich als Zernike-Polynom 2. Ordnung darstellen, so dass der Fokusfehler zu einem quadratischen Phasenfaktor führt. Aberrationen höherer Ordnung, wie sphärische Aberrationen oder Astigmatismus, können als entsprechend komplexere Funktionen dargestellt werden. Das erfindungsgemäße Verfahren kann so durchgeführt werden, dass nicht nur eine Abbildung in die falsche Bildebene, sondern auch Aberrationen höherer Ordnung korrigiert werden. Insbesondere kann ein Astigmatismus korrigiert werden.

In der paraxialen Näherung werden die sphärischen Wellenfronten, die von einem Objektpunkt ausgehen durch eine parabolische Wellenfront approximiert. Entsprechend lassen sich die parabolischen Wellenfronten durch einen quadratischen Phasenfaktor in eine plane Wellenfront oder auch eine konvergente Wellenfront eines beliebigen Radius umwandeln. Wenn der quadratische Phasenfaktor zu klein oder zu groß ist, wird das Objekt nicht korrekt in die Bildebene abgebildet. Die Strahlen eines Objektpunkts treffen sich dann vor oder hinter der Bildebene. Entsprechend erscheint der Objektpunkt in der Bildebene verwaschen.

Da bei der Full-Field-OCT nicht nur die Intensitäten, sondern auch die Phasen gemessen werden, hat man die Möglichkeit, die sich aus dem Fokusfehler ergebenden Phasenfehler numerisch zu beseitigen. Die Bilddaten werden dazu Fourier-transformiert und der Phasenfaktor wird im Fourierraum hinzugefügt. Das so veränderte Spektrum wird dann zurück in den Ortsraum transformiert, in dem man ein refokussiertes (scharfes) Bild des Objekts erhält. Mit diesen Schritten kann ein fokuskorrigiertes Bild des Augenhintergrunds berechnet werden.

Im einfachsten Fall ist die Fehlsichtigkeit des Patienten vorbekannt. Dann lässt sich aus dem Sehfehler in Dioptrien der zur Korrektur benötigter Phasenfaktor direkt bestimmen. Für den Defokus ist dies der quadratische Phasenfaktor $\Phi_{Defokus}$, der sich für den Radius $r_{Apertur}$ durch

$$\Phi_{Defokus}(i,j) = \frac{D \cdot \pi \cdot r_{Apertur}^2}{\lambda}$$

berechnen lässt. Dabei ist D die Fehlsichtigkeit in Dioptrien und A die zentrale Wellenlänge des Messsystems. Die erfindungsgemäße Berechnung eines fokuskorrigierten Bild des Augenhintergrunds kann demnach erleichtert werden, indem ein bei der Korrektur verwendeter Phasenfaktor aus dem vorbekannten Sehfehler des Patienten abgeleitet wird.

Nach der Aufnahme von Bilddaten mit dem Bildsensor kann demnach eine Berechnung vorgenommen werden, mit der aus den Bilddaten ein fokuskorrigiertes Bild des Augenhintergrunds berechnet wird. Die Berechnung kann in einer von dem Aufnahmegerät entfernten Recheneinheit durchgeführt werden. Die Bilddaten können über eine Datenverbindung an die Recheneinheit gesendet werden.

Die rechnerische Beseitigung eines Fokusfehlers setzt voraus, dass die Phasenlage während der Aufnahme des zu korrigierenden Bilds erhalten bleibt. Bei lebenden Proben geht die Phasenbeziehung durch die Bewegungen der Probe innerhalb kurzer Zeit verloren. Für den Augenhintergrund beispielsweise gilt, dass die Phasenlage typischerweise nur wenige 100 µs erhalten bleibt. Bei dem erfindungsgemäßen Verfahren kann die Aufnahme eines vollständigen Bilds aus der Objektebene (En-face Schnitt) innerhalb eines Zeitraums von weniger als 800 µs, vorzugsweise weniger als 500 µs, weiter vorzugsweise weniger als 300 µs erfolgen. Insbesondere kann das vollständige Bild innerhalb einer einzigen Belichtungszeit des Bildsensors erfasst werden.

Der Bildsensor kann eine Vielzahl von Pixeln umfassen, die beispielsweise in Form eines rechteckigen Arrays angeordnet sein können. Die Anzahl von Pixeln in einer Dimension des Rechtecks kann beispielsweise zwischen 500 und 5000, vorzugsweise zwischen 100 und 3000 liegen. Die Breite eines einzelnen Pixels kann beispielsweise zwischen 1 µm und 8 µm, vorzugsweise zwischen 2 µm und 5 µm liegen. Die Lichtquelle, mit der das kurzkohärente Licht erzeugt wird, kann beispielsweise eine Superlumineszenzdiode sein.

Das Verfahren kann so durchgeführt werden, dass die im Objektstrahlengang angeordneten optischen Elemente der verwendeten Vorrichtung starr sind. Mit anderen Worten ist es im normalen Betrieb der Vorrichtung nicht möglich, die Bildebene durch Justieren von optischen Elementen der Vorrichtung im Objektstrahlengang zu verschieben. Eine außerhalb des normalen Betriebs stattfindende Justierung beispielsweise bei der Herstellung oder Wartung der Vorrichtung ist dadurch nicht ausgeschlossen. Wenn auf die Möglichkeit einer Justierung verzichtet wird, wird die Durchführung von Messungen durch ungeschultes Personal erleichtert.

Um dem Patienten eine Orientierung für die Blickrichtung während der Aufnahme zu geben, kann ein Fixierlicht auf das Auge des Patienten gerichtet werden. Der Patient kann in Richtung des Fixierlichts blicken und damit eine für die Aufnahme geeignete Blickrichtung einnehmen.

Wenn bei dem erfindungsgemäßen Verfahren darauf verzichtet wird, den Objektstrahlengang in der Bildebene zu fokussieren, so kann dies Auswirkungen auf das Fixierlicht haben, indem das Fixierlicht für den Patienten nicht scharf sichtbar ist. Für das erfindungsgemäße Verfahren kann es deswegen von Vorteil sein, ein fokusunabhängiges Fixierlicht zu verwenden. Als fokusunabhängig wird ein Fixierlicht bezeichnet, wenn eine scharfe Wahrnehmung des Fixierlichts möglich ist, ohne dass eine Bildebene des Fixierlichts in Übereinstimmung mit der Netzhaut des Patientenauges gebracht wird. Ein Beispiel für ein fokusunabhängiges Fixierlicht ist ein Laserstrahl, der mit kleinem Durchmesser auf das Auge gerichtet wird. Ein solcher Laserstrahl wird vom Auge immer als scharfer Fleck wahrgenommen unabhängig davon, welchen Abstand zu der Lichtquelle das Auge hat. Es gibt andere Möglichkeiten, ein fokusunabhängiges Fixierlicht zu erzeugen, beispielsweise in dem das Licht von einer Lichtquelle durch eine konische Linse (Axicon) oder einen Hohlzylinder geleitet wird.

Ein Verfahren, bei dem ein fokusunabhängiges Fixierlicht verwendet wird, hat eigenständigen erfinderischen Gehalt, auch wenn darauf verzichtet wird, den Referenzstrahlengang vor dem Auftreffen auf den Bildsensor durch ein optisches Korrekturelement hindurchtreten zu lassen, um eine chromatische Abweichung innerhalb des Referenzstrahlengangs zu vermindern. Im Unterschied zu üblichen Messverfahren am Auge, bei denen sowohl die Messung an sich als auch die scharfe Wahrnehmung des Fixierlichts voraussetzen, dass der Patient die Blickrichtung und den Fokus richtig einstellt, kann der Patient sich bei diesem Verfahren alleine auf die Blickrichtung konzentrieren. Die Erfindung hat erkannt, dass damit Messfehler vermieden werden können, die sich daraus ergeben, dass der Patient sich gleichzeitig auf die Blickrichtung und den Fokus korrigieren soll. Es hat sich gezeigt, dass Patienten, die Schwierigkeiten mit der richtigen Einstellung des Fokus haben, auch zu Änderungen der Blickrichtung neigen. Diese Patienten haben es nun leichter, weil eventuelle Schwierigkeiten mit der Fokuseinstellung wegfallen.

Die Erfindung betrifft außerdem ein Full-Field-OCT-System mit einem Aufnahmegerät und einer Recheneinheit. Das Aufnahmegerät umfasst eine Lichtquelle zum Aussenden von kurzkohärentem Licht, und einen Strahlenteiler, um das kurzkohärente Licht in einen Objektstrahlengang und eine Referenzstrahlengang aufzuspalten. Der Objektstrahlengang wird zu einer Austrittsöffnung des Aufnahmegeräts geleitet. Auf einem Bildsensor werden der Referenzstrahlengang und ein von einem Objekt reflektierter Teil des Objektstrahlengangs zur Interferenz gebracht, wobei der Referenzstrahlengang unter einem von dem Objektstrahlengang abweichenden Winkel auf den Bildsensor trifft und wobei der Referenzstrahlengang vor dem Auftreffen auf den Bildsensor auf ein optisches Korrekturelement trifft, um eine chromatische Abweichung innerhalb des Referenzstrahlengangs zu vermindern. Die Recheneinheit ist dazu ausgelegt, aus mit dem Bildsensor aufgenommenen Bilddaten ein fokuskorrigiertes Bild des Objekts zu berechnen.

Das Aufnahmegerät des erfindungsgemäßen Systems kann insbesondere verwendet werden, um eine Abbildung eines hinteren Augenabschnitts eines menschlichen Auges zu erzeugen. Das Aufnahmegerät kann als Handgerät gestaltet sein, das dazu ausgelegt ist, von dem Patienten selbst gehalten zu werden, von dessen Auge die Abbildung aufgenommen wird. Das Handgerät kann so gestaltet sein, dass die optischen Elemente des Objektstrahlengangs im normalen Betrieb des Handgeräts nicht justiert werden können. Die Recheneinheit kann von dem Handgerät entfernt angeordnet sein. Es kann eine Datenleitung vorgesehen sein, über die Bilddaten von dem Handgerät zu der Recheneinheit übertragen werden können.

Das System kann mit weiteren Merkmalen fortgebildet werden, die im Zusammenhang des erfindungsgemäßen Verfahrens beschrieben sind. Das Verfahren kann mit weiteren Merkmalen fortgebildet werden, die im Zusammenhang des erfindungsgemäßen Systems beschrieben sind.

Die Erfindung wird nachfolgend unter Bezugnahme auf die beigefügten Zeichnungen anhand vorteilhafter Ausführungsformen beispielhaft beschrieben. Es zeigen:

Fig. 1:    eine schematische Darstellung eines erfindungsgemäßen Full-Field-OCT-Systems;

Fig. 2:    eine Darstellung der Strahlengänge des Handgeräts aus Fig. 1;

Fig. 3:    eine schematische Darstellung der Berechnung eines fokuskorrigierten Bilds;

Fig. 4:    einen anderen Aspekt des Handgeräts aus Fig. 2;

Fig. 5:    eine Ausführungsform eines fokusunabhängigen Fixierlichts.

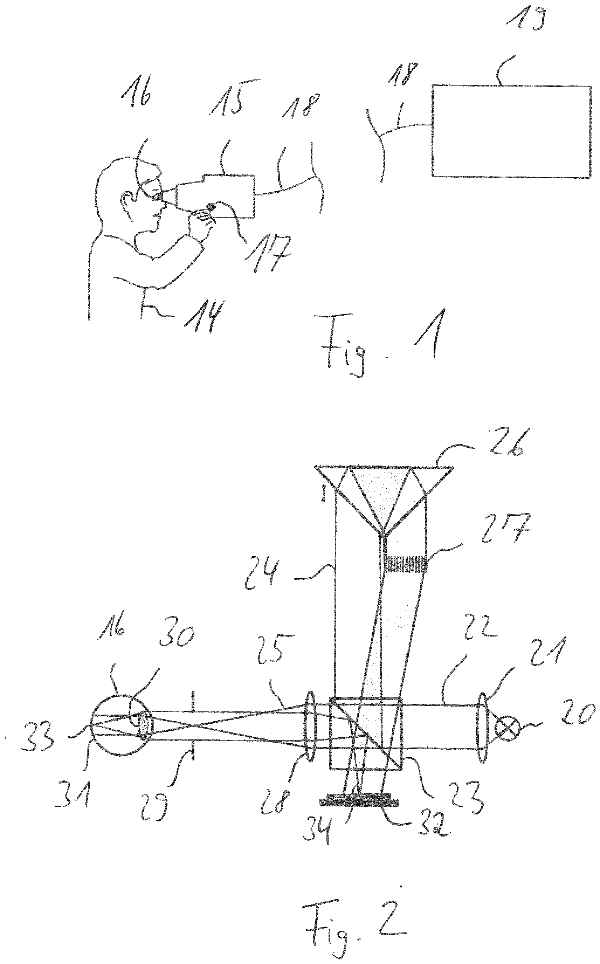

Bei dem in Fig. 1 gezeigten erfindungsgemäßen Full-Field-OCT-System hält ein Patient 14, von dessen Augenhintergrund eine Abbildung erzeugt wird, ein Handgerät 15 in der Hand. Das Handgerät 15 ist vor dem Auge 16 des Patienten 14 angeordnet, so dass der Patient 14 ein Fixierlicht im Inneren des Handgeräts 15 sehen kann. Nachdem der Patient 14 seine Blickrichtung anhand des Fixierlichts eingestellt hat, betätigt er einen Schalter 17, mit dem die Aufnahme einer Abbildung des Augenhintergrunds ausgelöst wird.

Nach Abschluss der Aufnahme werden die Bilddaten über ein Datennetzwerk 18 zu einem von dem Patienten 14 entfernten Zentralrechner 19 übertragen. Der Zentralrechner 19 kann beispielsweise in der Zentrale eines Dienstleisters angeordnet sein, der das Full-Field-OCT-System betreibt. Das System kann so eingerichtet sein, dass der Zentralrechner 19 Bilddaten von einer Vielzahl von Handgeräten 15 erhält, die an verschiedenen Orten oder in verschiedenen Einrichtungen betrieben werden. Insbesondere können die für eine gegebenenfalls erforderliche Fokuskorrektur erforderlichen Rechenschritte in dem Zentralrechner 19 vorgenommen werden. In alternativen Ausführungsformen der Erfindung ist das Handgerät 15 selbst dazu ausgelegt, die Rechenschritte für die Fokuskorrektur vorzunehmen.

Das Handgerät 15 umfasst gemäß Fig. 2 eine SuperlumineszenzDiode 20, die kurzkohärentes Licht mit einer Kohärenzlänge von beispielsweise 10 $\mu$m aussendet. Mit einer ersten Linse 21 wird ein kollimierter Strahlengang 22 des kurzkohärenten Lichts erzeugt, der auf einem Strahlenteiler 23 trifft. Mit dem Strahlenteiler 23 wird das kurzkohärente Licht 22 in einen Referenzstrahlengang 24 und einen Objektstrahlengang 25 aufgespalten.

Der Referenzstrahlengang 24 trifft auf ein Dachprisma 26, tritt durch eine Dachfläche in das Prisma 26 ein, wird an einer Hypothenusenfläche des Prismas 26 reflektiert und tritt durch die andere Dachfläche in entgegengesetzter Richtung, aber parallelverschoben wieder aus dem Dachprisma 26 aus. Der Referenzstrahlengang 24 trifft auf ein Transmissionsgitter 27 und wird durch das Transmissionsgitter 27 hindurch zurück in Richtung des Strahlenteilers 23 geleitet.

Der Objektstrahlengang 25 tritt ausgehend von der Superlumineszenz-Diode 20 durch den Strahlenteiler 23 hindurch und wird über eine zweite Linse 28 und eine Blende 29 auf das Auge 16 des Patienten geleitet. Der Objektstrahlengang 25 tritt durch die Augenlinse 30 hindurch in das Innere des Auges 16 ein und beleuchtet den Augenhintergrund 31 flächig.

Von dem Augenhintergrund 31 zurückgeworfene Anteile des kurzkohärenten Lichts 22 gelangen über die Augenlinse 30 und die zweite Linse 28 zurück zu dem Strahlenteiler 23, wo der Objektstrahlengang in Richtung eines Bildsensors 32 umgelenkt wird. Die optischen Elemente in dem Objektstrahlengang 25 sind so angeordnet, dass der Augenhintergrund 31 auf den Bildsensor 32 abgebildet wird. Dies ist in Fig. 2 am Beispiel eines Objektpunkts 33 dargestellt, der einem Bildpunkt 34 auf dem Bildsensor 32 entspricht.

Zwischen dem Strahlenteiler 23 und dem Bildsensor 32 ist der Objektstrahlengang 25 mit dem Referenzstrahlengang 24 überlagert. In der Ebene des Bildsensors 32 kommt es zu einer Interferenz zwischen dem Objektstrahlengang 25 und dem Referenzstrahlengang 24. Das Interferenzmuster wird mit dem Bildsensor 32 aufgenommen.

Beim Auftreffen auf den Bildsensor 32 schließen der Objektstrahlengang 25 und der Referenzstrahlengang 24 einen Winkel miteinander ein. Der Objektstrahlengang 25 trifft unter einem rechten Winkel auf den Bildsensor 32. Der Referenzstrahlengang 24 schließt beim Auftreffen auf den Bildsensor 32 einen Winkel von etwas weniger als 90° mit dem Bildsensor 32 ein, beispielsweise einen Winkel von 87°.

Die Richtung des Referenzstrahlengangs 24 wird durch das Transmissionsgitter 27 eingestellt. Vor dem Durchtritt durch das Transmissionsgitter 27 breitet der Referenzstrahlengang 24 sich in einer zu dem Bildsensor 32 rechtwinkligen Richtung aus. Mit dem Durchtritt durch das Transmissionsgitter 27 ändert der Referenzstrahlengang 24 seine Ausbreitungsrichtung. Dabei ist das Transmissionsgitter 27 so gestaltet, dass eine chromatische Abweichung vermieden wird. Mit anderen Worten ist das Transmissionsgitter 27 so gestaltet, dass die Pulsfront des aus dem Transmissionsgitter 27 auslaufenden Referenzstrahlengangs 24 parallel zu dem Bildsensor 32 ist. Die Pulsfront ist demnach nicht rechtwinklig zu der Ausbreitungsrichtung des Referenzstrahlengangs, sondern schließt mit der Richtung des Referenzstrahlengangs 24 einen anderen Winkel ein. Das Transmissionsgitter 27 bildet ein optisches Korrekturelement im Sinne der Erfindung, mit dem eine chromatische Abweichung innerhalb des Referenzstrahlengangs 24 vermindert wird.

Damit wird eine Verschmierung des von einem Objektpunkt 33 ausgehenden multispektralen Wellenfelds im Fourierraum vermieden. Dies gilt auch dann, wenn der Objektstrahlengang 25 anders als in Fig. 2 gezeigt nicht auf dem Bildsensor 32 fokussiert ist, sondern einen Fokusfehler aufweist. Die Berechnung des fokuskorrigierten Bilds kann demnach erfolgen, ohne dass das Resultat durch eine chromatische Verschmierung beeinträchtigt ist.

In Fig. 2 ist ein Objektstrahlengang 25 gezeigt, bei dem der Objektpunkt 33 durch die Augenlinse 30 ins Unendliche abgebildet wird. Das Auge 16 ist also frei von einer Fehlsichtigkeit. Das Handgerät 15 ist so justiert, dass sich eine fokussierte Abbildung auf dem Bildsensor 32 gibt, wenn das Auge 16 nicht fehlsichtig ist.

Bei einer Fehlsichtigkeit des Auges 16 ergibt sich ein Fokusfehler, bei dem die Bildebene nicht mit dem Bildsensor 32 übereinstimmt, sondern in einer Ebene vor oder hinter dem Bildsensor 32 liegt. Ein mit dem Bildsensor 32 aufgenommenes Bild erscheint verschwommen.

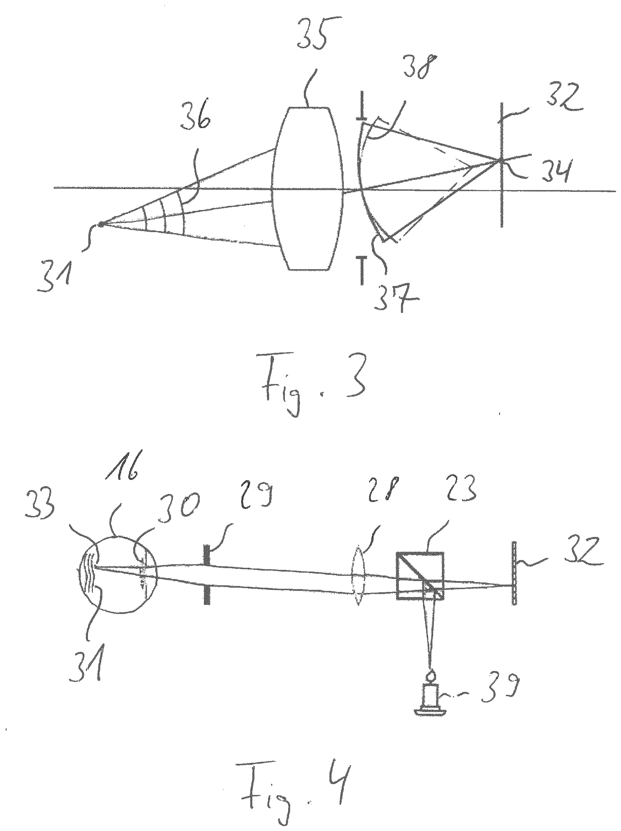

Die Schritte zur Korrektur des Fokusfehlers werden anhand von Fig. 3 erläutert. Jeder Objektpunkt 31 des Augenhintergrunds 33 kann als Ausgangspunkt einer divergenten Kugelwelle 36 betrachtet werden. Die divergente Kugelwelle 36 wird durch die Augenlinse 30 und die zweite Linse 28, die in Fig. 3 gemeinsam als ein Linsenbauteil 35 veranschaulicht sind, in eine konvergente Kugelwelle 37 umgewandelt. Bei einer nicht fehlsichtigen Augenlinse mündet die konvergente Kugelwelle 37 in einem Bildpunkt 34 auf dem Bildsensor 32 mündet.

Ist das Auge 16 fehlsichtig, so hat die konvergente Kugelwelle nach dem Linsenbauteil 35 nicht den richtigen Krümmungswinkel und der Bildpunkt liegt entweder vor dem Bildsensor 32 oder hinter dem Bildsensor 32. In Fig. 3 ist dies anhand einer konvergenten Kugelwelle 38 dargestellt, die zu stark gekrümmt ist und deren Bildpunkt vor dem Bildsensor 32 liegt.

Um bei einem solchen Fokusfehler ein fokuskorrigiertes Bild des Augenhintergrunds 33 zu erzeugen, werden die mit dem Bildsensor 32 aufgenommenen verwaschenen Bilddaten zunächst zu dem Zentralrechner 19 gesendet. Der Zentralrechner 19 unterzieht die Bilddaten einer Fourier-Transformation, so dass im Fourierraum ein geeigneter Phasenfaktor hinzugefügt werden kann. Das so veränderte Spektrum wird dann zurück in den Ortsraum transformiert, in dem man ein refokussiertes (scharfes) Bild des Objekts erhält. Da der Referenzstrahlengang 24 durch das Transmissionsgitter 27 chromatisch korrigiert wurde, kann die Korrekturrechnung für alle in dem kurzkohärenten Licht 22 enthaltenen Wellenlängen gemeinsam durchgeführt werden.

In Fig. 4 ist das Fixierlicht 39 des Handgeräts 15 dargestellt. Die Augenlinse 30 bildet den Objektpunkt 33 vom Augenhintergrund 31 ins Unendliche ab, das Auge ist also nicht fehlsichtig. Das Fixierlicht 39 ist so angeordnet, dass es von einem nicht fehlsichtigen Auge 16 scharf wahrgenommen wird.

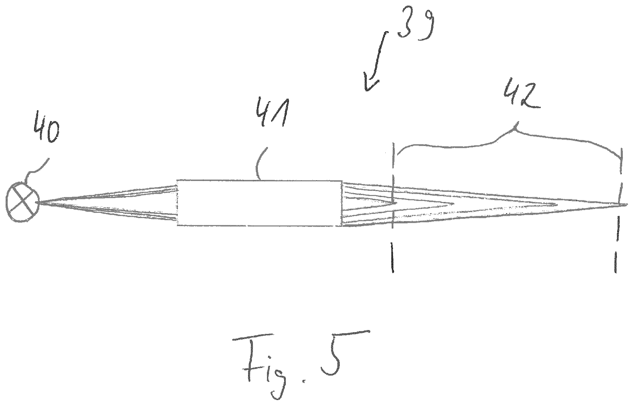

In Fig. 5 ist eine Ausführungsform eines Fixierlichts 39 gezeigt, das in unterschiedlichen axialen Positionen scharf wahrgenommen werden kann. Das von einer Lichtquelle 40 ausgesandte Licht wird in einem Hohlzylinder 41 geleitet, der so bemessen ist, dass jeder Lichtstrahl genau einmal im Inneren des Ausländers 41 reflektiert wird. Auf der anderen Seite des Hohlzylinders 41 ergibt sich ein axialer Abschnitt von der Länge 42, in dem das Licht als scharf begrenzter Fleck wahrgenommen werden kann. Dies ist ein Beispiel für ein fokusunabhängiges Fixierlicht 39, das auch von einem fehlsichtigen Patienten scharf wahrgenommen werden kann, ohne dass innerhalb des Handgeräts 15 optische Elemente justiert werden müssen.

## Patentansprüche

1. Full-Field-OCT-Verfahren zum Erzeugen einer Abbildung eines Augenhintergrunds (31) mit folgenden Schritten:

   a. Aussenden von kurzkohärentem Licht (22); von einer Lichtquelle (20);
   b. Aufspalten, durch einen Strahlenteiler (23), des kurzkohärenten Lichts (22) in einen Objektstrahlengang (25) und einen Referenzstrahlengang (24), wobei der Objektstrahlengang (25) auf den Augenhintergrund (33) geleitet wird;
   c. Leiten des Referenzstrahlengangs (24) und einen von dem Augenhintergrund (31) reflektierten Teil des Objektstrahlengangs (25) auf einen Bildsensor (32), so dass auf dem Bildsensor (32) eine Interferenz zwischen dem Referenzstrahlengang (24) und dem Objektstrahlengang (25) entsteht, wobei der Referenzstrahlengang (24) unter einem von dem Objektstrahlengang (25) abweichenden Winkel auf den Bildsensor (32) trifft und wobei der Referenzstrahlengang (24) vor dem Auftreffen auf den Bildsensor (32) auf ein optisches Korrekturelement (27) trifft, um eine chromatische Abweichung innerhalb des Referenzstrahlengangs (24) zu vermindern;
   d. Ermitteln, durch eine Recheneinheit (19), einer Intensitätsinformation und einer Phaseninformation aus einer Aufnahme des Bildsensors (32);
   e. Berechnen, durch die Recheneinheit, eines fokuskorrigierten Bildes des Augenhintergrunds (31). aus der ermittelten Intensitäts- und Phaseninformation.

2. Full-Field-OCT-Verfahren nach Anspruch 1, **dadurch gekennzeichnet, dass** die Richtung des Referenzstrahlengangs (24) mit einem Reflexionselement (26) umgelenkt wird.

3. Full-Field-OCT-Verfahren nach Anspruch 2, **dadurch gekennzeichnet, dass** der Referenzstrahlengang (24) vor dem Reflexionselement (26) von dem Referenzstrahlengang (24) nach dem Reflexionselement (26) separiert ist.

4. Full-Field-OCT-Verfahren nach einem der Ansprüche 1 bis 3, **dadurch gekennzeichnet, dass** die Länge des optischen Wegs des Referenzstrahlengangs (24) veränderbar ist.

5. Full-Field-OCT-Verfahren nach Anspruch 2 oder 3, **dadurch gekennzeichnet, dass** das optische Korrekturelement (27) zwischen dem Strahlenteiler (23) und dem Reflexionselement (26) angeordnet ist.

6. Full-Field-OCT-Verfahren nach einem der Ansprüche 1 bis 5, **dadurch gekennzeichnet, dass** das optische Korrekturelement (27) als Transmissionsgitter oder als Reflexionsgitter ausgebildet ist.

7. Full-Field-OCT-Verfahren nach einem der Ansprüche 1 bis 6, **dadurch gekennzeichnet, dass** das optische Korrekturelement (27) so eingestellt wird, dass die Normale der Pulsfront des aus dem optischen Korrekturelement (27) auslaufenden Referenzstrahlengangs (24) einen Winkel mit der Ausbreitungsrichtung des Objektstrahlengangs (25) einschließt, der kleiner ist als der Winkel zwischen der Ausbreitungsrichtung des Referenzstrahlengangs (24) und der Ausbreitungsrichtung des Objektstrahlengangs (25).

8. Full-Field-OCT-Verfahren nach einem der Ansprüche 1 bis 7, **dadurch gekennzeichnet, dass** ein bei der Berechnung des fokuskorrigierten Bilds verwendeter Phasenfaktor aus dem vorbekannten Sehfehler des Patienten abgeleitet wird.

9. Full-Field-OCT-Verfahren nach einem der Ansprüche 1 bis 8, **dadurch gekennzeichnet, dass** der Bildsensor (32) dazu ausgelegt ist, ein En-face-Schnittbild innerhalb eines Zeitraums von weniger als 800 μs, vorzugsweise weniger als 500 μs, weiter vorzugsweise weniger als 300 μs aufzunehmen.

10. Full-Field-OCT-Verfahren nach einem der Ansprüche 1 bis 9, **dadurch gekennzeichnet, dass** die im Objektstrahlengang angeordneten optischen Elemente (23, 28) starr sind.

11. Full-Field-OCT-Verfahren nach einem der Ansprüche 1 bis 10, **gekennzeichnet durch** ein Fixierlicht (39), das der Patient während der Aufnahme sieht.

12. Full-Field-OCT-Verfahren nach einem der Ansprüche 1 bis 11, **dadurch gekennzeichnet, dass** das Fixierlicht (39) ein fokusunabhängiges Fixierlicht ist.

13. Full-Field-OCT-System mit einem Aufnahmegerät (15) und mit einer Recheneinheit (19), das Aufnahmegerät (15) umfassend eine Lichtquelle (20) zum Aussenden von kurzkohärentem Licht (22), einen Strahlenteiler (23), um das kurzkohärente Licht (22) in einen Objektstrahlengang (25) und eine Referenzstrahlengang (24) aufzuspalten, wobei der Objektstrahlengang (25) zu einer Austrittsöffnung des Aufnahmegeräts (15) geleitet wird, und mit einem Bildsensor (32), auf dem der Referenzstrahlengang (24) und ein von einem Objekt reflektierter Teil des Objektstrahlengangs (25) zur Interferenz gebracht werden, wobei der Referenzstrahlengang (24) unter einem von dem Objektstrahlengang (25) abweichenden Winkel auf den Bildsensor (32) trifft und wobei der Referenzstrahlengang (24) vor dem Auftreffen auf den Bildsensor (32) auf ein optisches Korrekturelement (27) trifft, um eine chromatische Abweichung innerhalb des Referenzstrahlengangs (24) zu vermindern, und wobei die Recheneinheit (19) dazu ausgelegt ist, aus mit dem Bildsensor (32) aufgenommenen Bilddaten Intensitäts- und Phaseninformation zu ermitteln und aus diesen ein fokuskorrigiertes Bild des Objekts zu berechnen.

**Claims**

1. Full-field OCT method for generating an image representation of an ocular fundus (31), having the following steps:

   a. emitting short-coherent light (22) from a light source (20);
   b. splitting the short-coherent light (22) into an object beam path (25) and a reference beam path (24) by way of a beam splitter (23), wherein the object beam path (25) is guided onto the ocular fundus (33);
   c. guiding the reference beam path (24) and a part of the object beam path (25) reflected by the ocular fundus (31) onto an image sensor (32) such that interference between the reference beam path (24) and the object beam path (25) arises on the image sensor (32), wherein the reference beam path (24) is incident on the image sensor (32) at an angle that deviates from the object beam path (25) and wherein the reference beam path (24), prior to the incidence on the image sensor (32), is incident on an optical correction element (27) in order to reduce a chromatic deviation within the reference beam path (24);
   d. ascertaining intensity information and phase information from a recording of the image sensor (32) by way of a computation unit (19);
   e. calculating a focus-corrected image of the ocular fundus (31) from the ascertained intensity information and phase information using the computation unit.

2. Full-field OCT method according to Claim 1, **characterized in that** the direction of the reference beam path (24) is deflected using a reflection element (26).

3. Full-field OCT method according to Claim 2, **characterized in that** the reference beam path (24) upstream of the reflection element (26) is separated from the reference beam path (24) downstream of the reflection element (26).

4. Full-field OCT method according to one of Claims 1 to 3, **characterized in that** the length of the optical path of the reference beam path (24) is variable.

5. Full-field OCT method according to Claim 2 or 3, **characterized in that** the optical correction element (27) is disposed between the beam splitter (23) and the reflection element (26).

6. Full-field OCT method according to one of Claims 1 to 5, **characterized in that** the optical correction element (27) is embodied as a transmission grating or as a reflection grating.

**7.** Full-field OCT method according to one of Claims 1 to 6, **characterized in that** the optical correction element (27) is set such that the normal of the pulse front of the reference beam path (24) emerging from the optical correction element (27) encloses an angle with the propagation direction of the object beam path (25) that is smaller than the angle between the propagation direction of the reference beam path (24) and the propagation direction of the object beam path (25).

**8.** Full-field OCT method according to one of Claims 1 to 7, **characterized in that** a phase factor used when calculating the focus-corrected image is derived from the known refractive error of the patient.

**9.** Full-field OCT method according to one of Claims 1 to 8, **characterized in that** the image sensor (32) is designed to record an en-face section within a period of less than 800 $\mu$s, preferably less than 500 $\mu$s, with further preference of less than 300 $\mu$s.

**10.** Full-field OCT method according to one of Claims 1 to 9, **characterized in that** the optical elements (23, 28) disposed in the object beam path are rigid.

**11.** Full-field OCT method according to one of Claims 1 to 10, **characterized by** a fixation light (39) that the patient sees during the recording.

**12.** Full-field OCT method according to one of Claims 1 to 11, **characterized in that** the fixation light (39) is a focus-independent fixation light.

**13.** Full-field OCT system comprising a recording device (15) and comprising a computation unit (19), with the recording device (15) comprising a light source (20) for emitting short-coherent light (22), a beam splitter (23) for splitting the short-coherent light (22) into an object beam path (25) and a reference beam path (24), wherein the object beam path (25) is guided to an exit opening of the recording device (15), and comprising an image sensor (32), on which the reference beam path (24) and a part of the object beam path (25) reflected by an object are made to interfere, wherein the reference beam path (24) is incident on the image sensor (32) at an angle deviating from the object beam path (25) and wherein the reference beam path (24) is incident on an optical correction element (27) prior to the incidence on the image sensor (32) in order to reduce a chromatic deviation within the reference beam path (24), and wherein the computation unit (19) is designed to ascertain intensity and phase information from image data recorded with the image sensor (32) and to calculate therefrom a focus-corrected image of the object.

## Revendications

**1.** Procédé d'OCT plein champ permettant de produire une image d'un fond de l'œil (31), comprenant les étapes suivantes consistant à :

a. émettre une lumière à cohérence courte (22) à partir d'une source de lumière (20) ;

b. à l'aide d'un séparateur de faisceaux (23), scinder la lumière à cohérence courte (22) en une trajectoire de faisceau d'objet (25) et une trajectoire de faisceau de référence (24), la trajectoire de faisceau d'objet (25) étant dirigée sur le fond de l'œil (33) ;

c. diriger la trajectoire de faisceau de référence (24) et une partie de la trajectoire de faisceau d'objet (25) réfléchie par le fond de l'œil (31) sur un capteur d'image (32) de façon à créer sur le capteur d'image (32) une interférence entre la trajectoire de faisceau de référence (24) et la trajectoire de faisceau d'objet (25), dans lequel la trajectoire de faisceau de référence (24) est incidente sur le capteur d'image (32) selon un angle différent de la trajectoire de faisceau d'objet (25), et dans lequel la trajectoire de faisceau de référence (24) est incidente sur un élément correcteur optique (27) avant d'être incidente sur le capteur d'image (32) afin de diminuer une aberration chromatique à l'intérieur de la trajectoire de faisceau de référence (24) ;

d. déterminer, par une unité de calcul (19), une information d'intensité et une information de phase à partir d'une prise de vue du capteur d'image (32) ;

e. calculer, par l'unité de calcul, une image à mise au point corrigée du fond de l'œil (31) à partir des informations d'intensité et de phase déterminées.

**2.** Procédé d'OCT plein champ selon la revendication 1, **caractérisé en ce que** la direction de la trajectoire de faisceau de référence (24) est déviée par un élément de réflexion (26).

**3.** Procédé d'OCT plein champ selon la revendication 2, **caractérisé en ce que** la trajectoire de faisceau de référence (24) est séparée avant l'élément de réflexion (26) de la trajectoire de faisceau de référence (24) après l'élément de réflexion (26).

**4.** Procédé d'OCT plein champ selon l'une quelconque des revendications 1 à 3, **caractérisé en ce que** la longueur de la distance optique de la trajectoire de faisceau de référence (24) est variable.

**5.** Procédé d'OCT plein champ selon la revendication 2 ou 3, **caractérisé en ce que** l'élément correcteur optique (27) est disposé entre le séparateur de faisceaux (23) et l'élément de réflexion (26).

**6.** Procédé d'OCT plein champ selon l'une quelconque des revendications 1 à 5, **caractérisé en ce que** l'élément correcteur optique (27) est réalisé comme un réseau de transmission ou un réseau de réflexion.

**7.** Procédé d'OCT plein champ selon l'une quelconque des revendications 1 à 6, **caractérisé en ce que** l'élément correcteur optique (27) est réglé de telle sorte que la normale du front d'impulsion de la trajectoire de faisceau de référence (24) sortant de l'élément correcteur optique (27) forme un angle avec la direction de propagation de la trajectoire de faisceau d'objet (25) qui est inférieur à l'angle entre la direction de propagation de la trajectoire de faisceau de référence (24) et la direction de propagation de la trajectoire de faisceau d'objet (25).

**8.** Procédé d'OCT plein champ selon l'une quelconque des revendications 1 à 7, **caractérisé en ce qu'**un facteur de phase utilisé pour le calcul de l'image à mise au point corrigée est dérivé du défaut de vision préalablement connu du patient.

**9.** Procédé d'OCT plein champ selon l'une quelconque des revendications 1 à 8, **caractérisé en ce que** le capteur d'image (32) est conçu pour enregistrer une image en coupe de face dans un délai de moins de 800 µs, de préférence de moins de 500 µs, de plus grande préférence de moins de 300 µs.

**10.** Procédé d'OCT plein champ selon l'une quelconque des revendications 1 à 9, **caractérisé en ce que** les éléments optiques (23, 28) disposés sur la trajectoire de faisceau d'objet sont rigides.

**11.** Procédé d'OCT plein champ selon l'une quelconque des revendications 1 à 10, **caractérisé par** une lumière de fixation (39) que le patient regarde pendant la prise de vue.

**12.** Procédé d'OCT plein champ selon l'une quelconque des revendications 1 à 11, **caractérisé en ce que** la lumière de fixation (39) est une lumière de fixation indépendante de la mise au point.

**13.** Système d'OCT plein champ, comprenant un appareil de prise de vue (15) et une unité de calcul (19), l'appareil de prise de vue (15) comprenant une source de lumière (20) permettant d'émettre une lumière à cohérence courte (22), un séparateur de faisceaux (23) pour scinder la lumière à cohérence courte (22) en une trajectoire de faisceau d'objet (25) et une trajectoire de faisceau de référence (24), dans lequel la trajectoire de faisceau d'objet (25) est dirigée sur un orifice de sortie de l'appareil de prise de vue (15), et comprenant un capteur d'image (32) sur lequel une interférence est créée entre la trajectoire de faisceau de référence (24) et une partie de la trajectoire de faisceau d'objet (25) réfléchie par un objet, dans lequel la trajectoire de faisceau de référence (24) est incidente sur le capteur d'image (32) selon un angle différent de la trajectoire de faisceau d'objet (25), et dans lequel la trajectoire de faisceau de référence (24), avant d'être incidente sur le capteur d'image (32), est incidente sur un élément correcteur optique (27) afin de diminuer une aberration chromatique à l'intérieur de la trajectoire de faisceau de référence (24), et dans lequel l'unité de calcul (19) est conçue pour déterminer des informations d'intensité et de phase à partir de données d'image enregistrées par le capteur d'image (32) et pour calculer à partir de celles-ci une image à mise au point corrigée de l'objet.

Fig. 1

Fig. 2

Fig. 3

Fig. 4

Fig. 5

**IN DER BESCHREIBUNG AUFGEFÜHRTE DOKUMENTE**

**In der Beschreibung aufgeführte Patentdokumente**

- DE 102015113465 A1

**In der Beschreibung aufgeführte Nicht-Patentliteratur**

- Introduction to Fourier Optics. **J.W. GOODMANN.** Digital Holography. Springer, 2010